(19) **Europäisches Patentamt**
**European Patent Office**
**Office européen des brevets**

(11) **EP 1 804 705 B1**

(12) **EUROPEAN PATENT SPECIFICATION**

(45) Date of publication and mention
of the grant of the patent:
**15.09.2010 Bulletin 2010/37**

(51) Int Cl.:
**A61B 19/00** *(2006.01)*     **A61B 8/00** *(2006.01)*

(21) Application number: **05764042.7**

(86) International application number:
**PCT/EP2005/053490**

(22) Date of filing: **19.07.2005**

(87) International publication number:
**WO 2006/008300 (26.01.2006 Gazette 2006/04)**

(54) **APARATUS FOR NAVIGATION AND FOR FUSION OF ECOGRAPHIC AND VOLUMETRIC IMAGES OF A PATIENT WHICH USES A COMBINATION OF ACTIVE AND PASSIVE OPTICAL MARKERS**

GERÄT ZUR NAVIGATION UND FUSION VON ECOGRAPHISCHEN UND VOLUMETRISCHEN BILDERN EINES PATIENTEN UNTER VERWENDUNG EINER KOMBINATION VON AKTIVEN UND PASSIVEN OPTISCHEN MARKERN

DISPOSITIF POUR LA FUSION ET LA NAVIGATION RELATIVES A DES IMAGES ECOGRAPHIQUES ET VOLUMETRIQUES PAR L'UTILISATION COMBINEE DE MARQUEURS OPTIQUES ACTIFS ET PASSIFS

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.07.2004 IT MI20041448**

(43) Date of publication of application:
**11.07.2007 Bulletin 2007/28**

(73) Proprietor: **Politecnico Di Milano**
**20133 Milano (IT)**

(72) Inventors:
• **DELLACA', Raffaele**
**I-22100 COMO (IT)**
• **ALIVERTI, Andrea**
**I-22100 COMO (IT)**
• **PEDOTTI, Antonio**
**I-20129 MILANO (IT)**

(74) Representative: **Mittler, Enrico**
**Viale Lombardia 20**
**I-20131 Milano (IT)**

(56) References cited:
**US-A- 4 836 778**     **US-A1- 2001 007 919**
**US-A1- 2003 208 296**     **US-A1- 2004 138 556**
**US-B1- 6 374 135**

## Description

The present invention concerns an apparatus for fusion and navigation of ecographic and volumetric images of a patient which uses a combination of active and passive optical markers for localization of ecographic probes and surgical instruments with reference to the patient.

In the past years technological progress in the field of the acquisition of biomedical images has allowed to obtain more and more detailed information both on the morphology and on the functions of the different anatomical areas of a patient. In this context the fusion of the information obtained with different techniques for the acquisition of images is playing an increasingly important role, both in diagnostics and in programming and execution of surgical operations. The fusion of images coming from different technological approaches, in fact, allows to combine the specific advantages of each technique, while limiting their respective applicative constraints. In particular it seems to be very interesting the combination of systems for the acquisition of volumetric images (CAT, MRI, PET, etc.) which is capable to provide images with very high morphologic and functional details, with 2D or 3D ultrasonography, non-invasive, flexible technique and ideal instrument for support to the surgeon in the stages of planning and, above all, execution of surgical operations. The characteristics of the ecographic analysis allow the surgeon to obtain a verification of his work and, therefore, to organize and to execute an operation by adjusting in real time to the possible variations of the conditions of the subject. The recent introduction of contrast means in echography has further improved both the quality of the ecographic images and the possibility to extract information of functional type from such images. In any case, unfortunately, limits intrinsic to the ecographic technology do not allow to obtain very defined images for all the types of tissues and to localize anomalies with the same accuracy and evidence that other techniques for the acquisition of images offer. In this context a system capable to combine in real time ecographic images with 3D images previously obtained with other image acquisition techniques would be an important advance for the development of diagnostic and surgical techniques which offer a greater effectiveness and a lower impact on the patient, with consequent improvement of the quality of the life and reduction in the cost of the operation.

In order to create a complete system in support of diagnosis, planning and execution of operations by combining information obtained from 3D. images acquisition techniques with ultrasonographical techniques it is necessary to solve two important technological problems: 1) the fusion of images acquired in different moments and with different techniques (which inevitably create geometric distortions in the recontructed images); 2) the real time localization of the position of the ecographic probe with reference to the patient in order to be able to obtain the information necessary to the navigation and fusion systems which combine the 3D images obtained with other methods with the 2D ecographic ones so as to obtain a new image having a higher informative content.

In addition it would be very useful to be able to localize in space not only the ecographic probe but also some surgical instruments (for example needles for thermoablation, endoscopes, etc.) used in the mini-invasive surgery. In this way it would be possible to superimpose the position of the parts of the instrument inserted inside the body and therefore not visible from the outside to the ecographic and CAT images.

US-A-2004/0138556 disclosed an optical object tracking system including the features of the preamble of claim 1.

Object of the present invention is to provide a unit of support to diagnosis and to surgery that meets the requirements reported above.

According to the present invention such object is attained with an apparatus for fusion of ecographic and volumetric images of a patient and for navigation and localization of an ecographic probe and a surgical instrument operating on the same patient, as defined in claim 1.

The characteristics of the present invention will be better understood through the following detailed description of an embodiment thereof which is illustrated as a non-limiting example with reference to the enclosed drawings, in which:

Figure 1 shows a blocks diagramme of principle of an apparatus according to the invention as applied to a patient;
Figure 2 shows the magnified detail of an ecographic probe provided with active markers according to the present invention;
Figure 3 shows the magnified detail of a surgical instrument provided with active markers according to the present invention;
Figure 4 shows the circuit layout of a possible starting device for the illuminators and the active markers which is utilisable in the apparatus according to the invention.

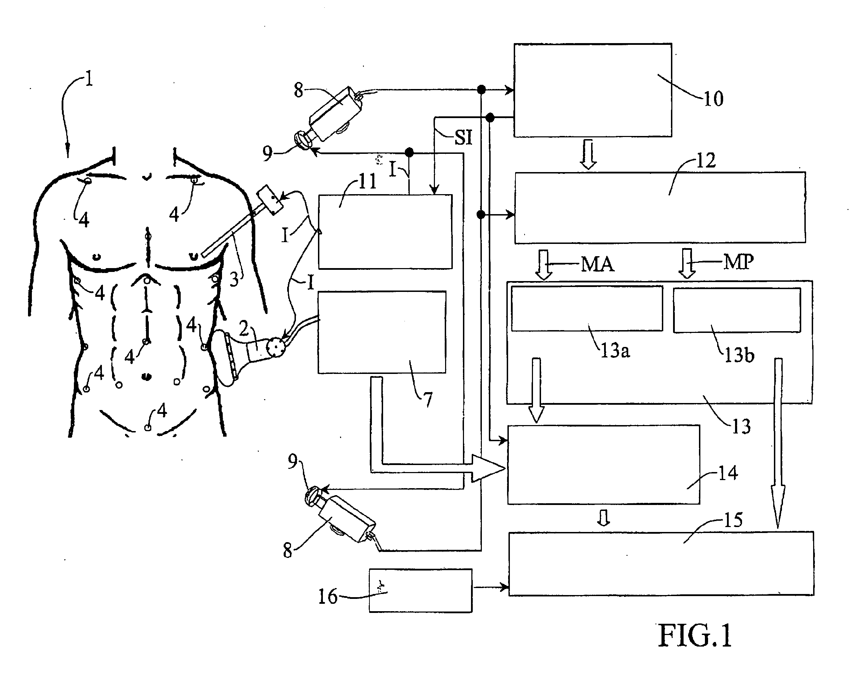

The apparatus shown in Figure 1 is capable to acquire images from the body of a patient, schematically represented and indicated by 1, on which an ecographic probe 2 and/or a surgical instrument 3 can operate.

Optical markers 4, preferably passive (that is capable to reflect appropriate optical signals), as for instance spherical or semispherical objects coated with back-reflecting materials are applied to the patient. The markers 4 get applied to the surface of the patient to correspond with detection or " repere" points in order to: a) localize the patient in the laboratory space and b) identify some useful detection points for the recording and the fusion of images acquired with other techniques for the formation of 3D images, as additionally explained hereinafter. For the scope a) at least three markers must be used. It is advisable to use a higher number since the body of the patient is not a rigid body but a relatively deformable structure. A higher number of markers allows therefore to better characterize the position of the different anatomical areas and, in addition, to consider also low deformations that occur during the measurement because of movements of the patient or the normal respiratory activities. For the scope b) too a minimum of three markers must be used, which can coincide with some or with all the ones in the scope a). The detection points utilised can be of two types, anatomical or artificial, which get applied to the body of the patient on the occasion of the acquisitions of 3D images carried out by different techniques. For example, in the case of fusion with CAT images, semi-permanent tattoos can be carried out on the skin of the subject to correspond with the position of radio-opaque markers present during CAT scan. Subsequently, before beginning the examination or operation procedures which utilises the apparatus according to the present invention, optical markers 4 are applied on the subject exactly to correspond with the tattooed signs.

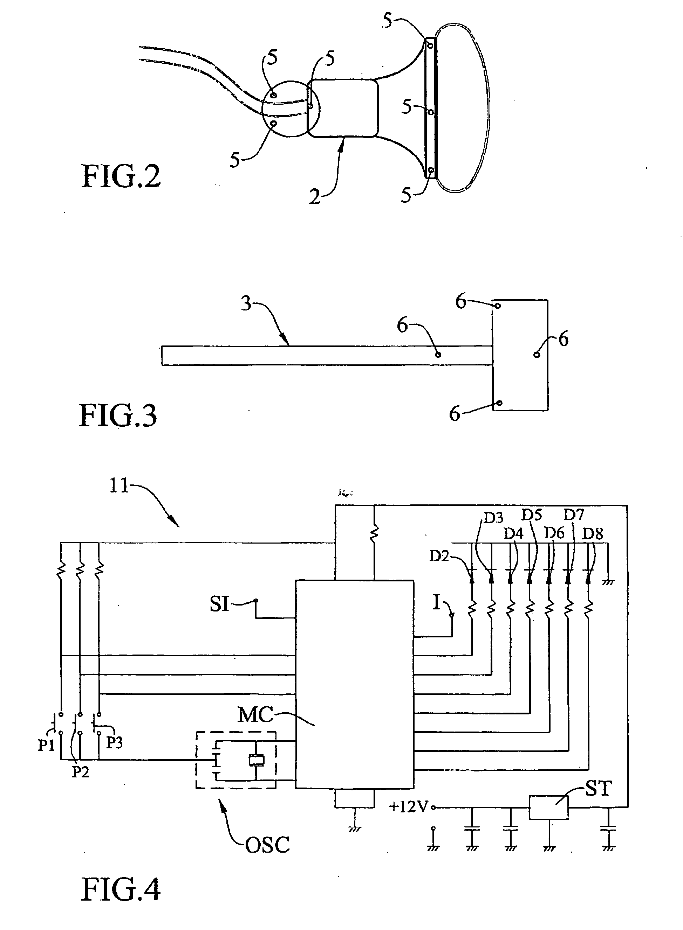

To the ecographic probe 2 and/or surgical instrument 3 active optical markers 5 and 6 (Figures 2 and 3), as for instance made up of infrared radiation LED diode valves with wide emission angle, are in turn applied. As an ecographic probe 2 a normal ecographic probe is utilisable which can be of convex or linear or endocavitary type, depending on the clinical applications. Such probe is modified by applying some (at least three) active markers 5, for instance arranged as in Figure 2. The use of a number of markers higher than three (the minimal number of points necessary to localize a rigid body in the space) allows to reconstruct the position of the probe even in the case in which some markers might not be visible for a few moments because, for example, covered by the hand of the physician who handles the probe. The ecographic probe 2 is connected with a device 7 for the acquisition of ecographic images, as for instance made up of a normal ecographer for clinical applications with an analogic or digital video output, which processes signals coming from the probe 2 in order to produce a two-dimensional image of the anatomical area under examination.

The surgical instrument 3 can in turn be provided with active markers 6 similar to the ones of the probe 2 (Figure 3).

Two or more optical sensors for example made up of video cameras 8 provided with devices for the activation of the passive markers 4, for example made up of illuminators 9 (as for instance infrared light LEDs) coaxially mounted with the objective of the video camera, receive the optical signals emitted by the active markers 5 and the ones reflected by the passive markers 4.

The video signal coming from each video camera 8 is received by a device 10 for the analysis of the movement, which processes them with the aim of extracting the two-dimensional coordinates of each marker 4, 5 or 6 present in each image. Starting from the two-dimensional coordinates of the markers and from the previously obtained calibration information it is possible, by applying opportune photogrammetry algorithms (as for instance the ones described in Borghese, N. A. and G. Ferrigno, An algorithm for 3-D Automatic Movement Detection by means of Standard TV Cameras, Ieee Trans Biomed Eng 37: 1221-1225, 1990), to obtain the accurate measure of the position of the markers in a reference system common to the laboratory. The markers recognized by the system can be both passive and active. In the case of the passive markers the light generated by the illuminators 9, being reflected by the markers, gets detected by the video cameras 8. In the case of the active markers, the light detected by the video cameras 8 is instead generated directly by the marker. To the purpose of reducing the interferences with the atmosphere where the measurement is carried out, it is preferable to use radiations different from the visible ones, in particular infrared light. In this case the active illuminators and/or markers are made up of sets of high luminosity LED diode valves which produce infrared radiation and the video cameras are provided with filters which allow the passage of the infrared radiation, therefore, attenuating the visible light In order to implement the present application it is possible to adopt optoelectronic analyses devices available on the market, upon condition of being able to modify the timing logics for the turning on of the illuminators since the unit according to the invention requires the possibility to turn on and to turn off both the illuminators and the active markers in independent and coordinate way, as described hereinafter.

The simultaneous presence of markers on the surface of the patient and on the ecographic probe 2 and/or on the surgical instrument 3 operated by the physician can easily create problems in the calculation of the position of the markers in the space because of the difficulties in solving the problem known as "stereo matching". In order to improve the reliability of the system for the calculation of the position of the markers placed on the probe and of the ones placed on the patient passive markers are used on the subject (which do not require connection cables, are simple to apply and can be single-use) and active markers on the probe. The turning on of the active markers and of the illuminators are managed by a synchronized starting device 11 in such a way so as to have the active markers turned on only when the illuminators are turned off. The illuminators 9 are therefore turned on only on the occasion of a subset of the image pictures acquired by the device 10. For example, if the device 10 for the analysis of the movement acquires at the frequency of 60 pictures per second, the illuminators 9 could be alternatively turned on (a picture on and a picture off). In this way the images of the markers applied to the subject and of the ones applied on the probe are obtained as alternate pictures. In addition, if one wants to have a higher sampling frequency for localization of the probe as compared with the one used for the localization of the patient (which is supposed to move much slower than the ecographic probe), it is possible to change the turning on sequences of the illuminators and of the active markers, as for instance by maintaining the active markers turned on in order to have more pictures so as to then turn them off and to turn the illuminators on for a single picture. The device 11 receives the necessary synchronisms from the central processing unit of the device 10 for the analysis of the movement and from it the controls for the turning on of the illuminators and of the active markers start. In the simplest of the embodiments the device 11 can for instance be made up of simple digital circuits in cabled logics (for example a Johnson counter with the outputs opportunely connected by a matrix of diode valves). The use of integrated microcontrollers, instead, allows to opportunely codify the models for the turning on of the active markers, thus facilitating their classification as described hereinafter.

A possible implementation of the turning on device 11 is represented by the circuit in Figure 4. A microcontroller MC (as for instance a 20 Mhz PIC 16F876 microchip) recognizes synchronism signals generated at the acquisition of each picture by the device 10 for the analysis of the movement. Once the acquisition being made has been recognized, the microcontroller MC activates the corresponding outputs either to the illuminators of the video cameras (signal I in Figure 4) or to the active markers (LEDs D2-D8 in Figure 4) that one wants to be turned on. The video cameras 8 will thus carry out the new acquisition in the conditions preset by the microcontroller MC. The device 11 must in addition provide the subsequent analysis blocks (described hereinafter) with a signal indicating if in a certain picture the information contained concerns the position of the passive markers placed on the patient (illuminator turned on and active markers turn offed) or the position of the active markers placed on the probe (illuminator turned off and active markers turned on). Such signal, for example, could consist in the same signal used in order to turn the illuminators 9 on. The presence of one or more surgical instrument provided with active markers is managed by alternatively turning on the active markers placed on the different instruments and the ecographic probe. In this way flows of data N are obtained identified by N equal to the number of objects to be localized (which represents the position of the passive markers applied on the patient). In this way any possible interference in the reconstruction of the 3D position of the markers is therefore prevented. Three push-buttons P1, P2 and P3 with associated OSC quartz oscillator allow to configure the model for the turning on of the illuminators and of the active markers according to the needs of the user. By ST a tension stabilizer is indicated in Figure 4.

The flow of data coming from the analysis device 10 is processed by a decoding device 12 in such a way so as to possibly separate the coordinates of the active markers on the ecographic probe 2 (and on the surgical instrument 3) from those of the active markers placed on the body surface of the patient. An opportune program, being the information relative to the turning on sequence of illuminators and active markers known, decodes the flow of data coming from the device 10 so as to obtain two (or more, in the case of markers placed also on one or more surgical instruments) distinct sequences MP and MA, the first containing the 2D coordinates of the passive markers for each moment of turning on of the illuminators, the second (and possibly the other ones, in the case of the presence of surgical instruments) containing the 2D coordinates of the active markers for each moment of turning on of the LED. Once the time sequences of the coordinates of the active and passive markers have been subdivided, subsequent programs will therefore be capable to calculate the 3D coordinates and to identify automatically and with a minimum probability of error the different markers.

The calculation of the three-dimensional coordinates of the markers is based on stereo-photogrammetry algorithms, which require the valuation, carried out previously, of the position, of the orientation and of the geometric parameters which identify the optical characteristics of the different video cameras in the reference system of the laboratory, for instance as described in Cerveri, Borghese, & Pedotti, 1998, Complete calibration of a stereo photogrammetric system through control points of unknown coordinates. Journal of Biomechanics, 31, 935-940; Tsai, 1987, A versatile camera calibration technique for high accuracy 3D machine vision metrology using off-the-shelf TV cameras and lenses. IEEE Journal of Robotics and Automation, RA-3(4), 323-344.

Opportune "tracking" procedures are instead utilised in order to be able to follow the movement of the markers in real time, while maintaining the information on their classification. Such procedures can be based:

a) on prediction algorithms bases on analytical criteria (as for example, Gamage & Lasenby, 2002, New least squares solutions for estimating the average center of rotation and the axis of rotation, Journal of Biomechanics, 35, 87-93) or functional criteria (as for example, Cappozzo, Catani, Leardini, Blessed, & Groce, 1996, Position and orientation in space of bones during movement: Experimental artifacts. Clinical Biomechanics, 11, 90-100).

b) on prediction algorithms bases on the Kalman filtering (as for example, Brown & Hwang, 1997, Introduction to random signals and applied Kalman filtering (3rd ed.), New York: John Wiley & Sons Inc.), which for instance determines the temporal series of the coordinates of the different markers by means of a picture by picture minimization of the distances between the points as measured by the video cameras and the corresponding back-projected model (for example, Cerveri, Pedotti, Ferrigno, Robust recovery of human motion from video using Kalman filters and virtual humans Human Movement Science 22 (2003) 377-404). In the case of the markers placed on the probe, the model is the one of a rigid body with six degrees of freedom, in which the relative distances between markers are known. In the case of the markers placed on the patient, the model is instead the one of a deformable body, in which strong restraints are anyhow present on the distances of the markers and on their movement.

The two sequences of data MA and MP, respectively relative to the active markers and the passive markers, are received by a data processing device 13, which can be considered as being subdivided in two distinguished parts 13a and 13b, respectively for localization of the ecographic probe and/or of the surgical instrument and for the definition of the movement of the patient.

It is a device capable to process the data coming from the decoding device 12, relative to the space coordinates of the active markers placed on the echography probe and possibly on the surgical instrument and of the back-reflecting passive markers placed on the patient, with the aim of localizing probe, image plane and patient in a single absolute reference system L (laboratory), determined by a set of cartesian axes $X_L$, $Y_L$, $Z_L$.

The device 13 identifies with opportune processing methods the parameters of the geometric transformations (roto-translations) to be carried out on the points belonging to the plane of the ecographic image and it calculates the new co-ordinate of such points in the reference system of the laboratory according to the equation

$$P'(x_L, y_L, z_L, t) = T(t) \cdot C \cdot P(x_1, y_1, t) \qquad (1)$$

where $P(x_1, y_1, t)$ is any point belonging to the image plane, variable in time t during the image scanning, C defines the constant of geometric transformation between the reference system S relative to the image plane and the reference system S integral with the probe, T(t) defines the geometric transformation between S and L, P' $(x_L, y_L, t)$ represents each point represented in the reference system L.

The previous equation can be expressed in matrix form:

$$\begin{bmatrix} P' \\ 1 \end{bmatrix} = \begin{bmatrix} R_T & O_T \\ Z_T & 1 \end{bmatrix} \cdot \begin{bmatrix} R_C & O_C \\ Z_C & 1 \end{bmatrix} \cdot \begin{bmatrix} P \\ 1 \end{bmatrix} \qquad (2)$$

where R stands for the rotation sub-matrixes (made up of director cosines) and O for the translation or offset sub-matrixes, whereas Z for vectors of zeros.

While the identification of matrix C is predetermined by an opportune calibration, the matrix T, variable in time, is identified at each moment of measurement.

The different calibration methods which can be utilised for the determination of the matrix C can be brought back to three different categories: 1) single point (or single line); 2) 2-D alignment; 3) "freehand" methods.

Single point methods use a calibration object ("phantom") that contains a target point ("target") made up of a sphere, a grain or a pin (for example, Legget et al., System for quantitative three-dimensional echocardiography of the left ventricle based on a magnetic-field position and orientation sensing system. IEEE Trans Biomed Eng, 1998, 45: 494-504) or a cross-wire (for example, Barry et al., Three-dimensional freehand ultrasound: Image reconstruction and volume analysis. Ultrasound Med Biol, 1997; 23: 1209-1224). The target is visualized from different directions. The advantage of these methods is their semplicity, even if the number of images to be acquired must be higher than the number of possible degrees of freedoms (three rotations and three translations). Typically, a few tens of images are used for the calculation of C. A variation of this approach considers the possibility to visualize a planar structure by means of an image plane US approximately perpendicular to the same structure (for example, Hook 2003, Probe calibration for 3D ultrasound image localization. Internship report. Laboratoire TIMC Grenoble, France / University of Magdeburg, Germany), thus obtaining a line which can be easy identified. Specific points on different images are then utilised for the calibration.

The idea at the basis of the 2-D alignment methods, instead, is to manually align the image plane US with a planar set of points (for example, cross-wire or tips of toothed membranes), using as a guide and reference the display of the ecographer (for example, Berg et al., Dynamic three-dimensional freehand echocardiography using raw digital ultrasound data. Ultrasound Med Biol, 1999, 25: 745-753). Since the points are distributed on a 2-D plane, the orientation of the plane is not ambiguous and, in principle, only one image is necessary for the calibration. However, since such plane has in fact a finished thickness, the alignment procedure can be as a result long and difficult, and the result can be not satisfactory in terms of accuracy.

In the recent past different calibration methods have been proposed which use "free-hand" scannings (for example, Bouchet et al., Calibration of threedimensional ultrasound images for image-guided radiation therapy. Phys Med Biol, 2001, 46: 559-577). The calibration objects utilised are made up of wires arranged according to certain configurations which allow, by observing the different images, a univocous determination of the position and of the orientation of the image plane US. In other words, such methods do not require burdensome alignment procedures with points, lines or planes, and only a few images are necessary. Such procedure well adjusts to clinical conditions, in which it is not always possible to avail of long times and skilled staff.

As per what concerns instead the identification of the matrix T, different methods can be implemented, the simplest one of which is the so-called Gram-Schmidt orthonormalization. Such procedure, having available the coordinates of three active markers integral with the probe, reconstructs three axes $w_1$, $w_2$, $W_3$ as it follows:

$$w_1 = \begin{bmatrix} x_A - x_O \\ y_A - x_O \\ z_A - x_O \end{bmatrix} \qquad w_2 = \begin{bmatrix} x_B - x_O \\ y_B - x_O \\ z_B - x_O \end{bmatrix} \qquad w_3 = w_1 \wedge w_2 \qquad (3)$$

where by O the marker chosen as origin is meant, whereas A ($x_A$, $y_A$, $z_A$) and B ($x_B$, $y_B$, $z_B$) are the other two markers. Starting from such axes, the algorithm therefore reconstructs an orthogonal base $u_1$, $u_2$, $u_3$:

$$(u_1 = w_1, u_2 = w_2 - K \cdot u_1, u_3 = w_3), \qquad \text{con } K = \frac{w_2 \cdot u_1}{u_1 \cdot u_1}.$$

The orthonormal base is finally obtained by dividing $u_1$, $u_2$, and $u_3$ by their norm. The three column vectors of the orthonormal base make up the elements of the rotation matrix $R_T$ of the equation 2, whereas the vector $O_T$ is made up of the coordinates of the marker O.

As an alternative to the method previously described, in order to identify the reference system of the probe, the so-called "redundant" methods can be used which uses a number of markers higher than three. In this way it is possible not only to obtain with higher accuracy the roto-translation parameters, but also to manage possible occlusions of the markers which can occur during the acquisition.

The processing device 13, in addition, utilises the coordinates of the passive markers placed on the patient with the aim of localizing the reference system of the patient in the reference system of the laboratory, with methods similar to the ones previously described. In the case in which one utilises markers detectable both by the device 10 and by other systems for the acquisition of volumetric images (such as CAT, MRI, PET, etc.), as for instance radio-opaque spheres coated with back-reflecting material, and such markers are placed in the same positions as chosen previously, the determination of the reference system of the patient allows the fusion of the different images.

The apparatus in Figure 1 comprises in addition a device 14 for the acquisition of the ecographic images in synchronism with the position of the ecographic probe 2 and/or of the surgical instrument 3. It consists of a device capable to receive as an input the sequence of images coming from the ecographer 7 and to record them in a synchronous way with the turning on of the illuminators and of the active markers placed on the probe and/or on the surgical instrument. The device 14 can be made up of an opportune video signal digitalizer installed on a personal computer, controlled by a program which receives as an input (for instance through a serial or parallel port) synchronization signals coming from the device 10 and which sends opportune control signals to the video digitalizer for the acquisition and the digitalization of the images. For example, the program could be structured in such a way so as to carry out the acquisition of the images that are necessary only at the moments of the turning on of the active markers placed on the ecographic probe so as to be able to reconstruct in the reference system of the patient each point of the ecographic image and to provide the set of the geometric parameters that identify the localization of the plane to which the same image belongs. The program in addition can be structured in such a way so as to define in parametric form the resolution of the image to be acquired (number of pixels per line and per column), the temporal solution (images per second) and possibly the region of interest of the video image provided by the ecographer 7.

Finally the apparatus in Figure 1 includes a device 15 for the fusion and the navigation of the ecographic images with volumetric images coming from other systems for the acquisition of images, generically indicated by the block 16.

The fusion between ecographic images and volumetric images such as CAT, MRI, PET, etc. turns out to be possible when, at the moment of the recording of the volumetric images, opportune detection points have been acquired and subsequently, during the surgical session or the ecographic analysis, passive markers detectacle by the video cameras 8 are applied to correspond with such points. In these conditions the device 15, provided with an opportune calculation and visualization program, will be capable to present to the operator in real time both the volumetric images as well as the ecographic images, represented in the same reference system, identified by the device 13. At each instant of acquisition of the ecographic images, the device 15, starting from the set of the geometric parameters coming from the device of acquisition 14 and from the data relative to the position of the patient coming from the data processing device 13, carries out the following calculations: a) it calculates the position of each element of the ecographic image in the reference system of the patient; b) it calculates the position of each element of the volumetric image in the reference system of the patient; c) it represents on a screen, in a single spacial reference, the volumetric images (represented by means of sections chosen by the operator), the ecographic images and possibly the surgical instrument.

In conclusion, the apparatus in Figure 1 allows to measure and to monitor in real time the position in space of detection points identified on the patient. At the moment of the recording of the volumetric images (such as CAT, MRI, PET, etc.) such detection points can consist both in anatomical references as well as in opportune objects of identification located generically on the body surface and detected by the systems for the acquisition of volumetric images (for example, radio-opaque spheres during the CAT scan). Subsequently, during the surgical session or the ecographic analysis, spherical or semispherical objects coated with back-reflecting materials ("markers") detectable by the opto-electronic system for the analysis of the movement are applied to correspond with such points. The position in space of such markers is therefore used as a reference system for the localization of the patient and for the alignment and the fusion of the images obtained through the different techniques for the acquisition of volumetric images. By applying an opportune number of active optical markers (for example, LED diode valves) on supports rigidly bound with the ecographic probe operated by the physician and/or with the surgical instrument it is also possible to identify the 3D position of the same probe and/or of the instrument and, therefore, to fuse the ecographic images with the ones obtained with the other techniques and to superimpose such images with the image of the instrument.

Particularly critical in the apparatus herein described is the stage of localization of the markers applied on the probe and/or on the instrument and of the markers applied on the subject. In fact by moving the probe and/or the instrument on the body surface there is the risk that the images of the markers located on the subject can be fused or in any case confused with the ones of the markers on the probe in the stage of 3D reconstruction. In order to improve the ability to discriminate the markers of the probe and/or of the instruments from the ones placed on the subject one synchronises, as already said, the turning on of the active markers with the system for the analyses of the movement through opportune devices (11) capable to turn the illuminators 9 on which activate the passive markers alternatively to the markers on the probe or to the ones on the instruments. In this way two or more separate flows of data are obtained, one relative to the position of the detection points which identify the position of the patient, one associated with the coordinates relative to the active markers which localize the probe, and, possibly, one or more flows of data which localize as many surgical instruments, preventing, therefore, possible calculation errors. In addition, the use of determined codes for the turning on of the active markers located on the probe and/or on the tools allows to automate the classification procedures necessary to the 3-D calculation. The coordinates of a few markers opportunely applied on the patient allow in addition to obtain useful information on his conditions, for example, the stage of the respiratory activity and the possible deformations induced on the chest by the variations of position with reference to the one assumed during the recording of the images obtained with the other methods. The methods proposed in this patent allow to obtain advanced ecographic systems for: 1) the fusion of the ecographic images with other techniques for the acquisition of volumetric images; 2) the support to surgery, by allowing the simultaneous recording, in the same reference system, of the positions of the patient, of ecographic probe and surgical tools, by compensating possible movements of the same patient. In particular, through the synchronized detection of the ultrasonographic images and through their fusion with pre-surgical images the identification of anatomicsl-functional elements evidenced by techniques such as CAT, MRI and contrast means US becomes possible thus facilitating and optimizing the localization and navigation of anatomical areas and their surgical treatment.

## Claims

1. Apparatus for fusion of ecographic and volumetric images of a patient (1) and for navigation and localization of an ecographic probe (2) and a surgical instrument (3) operating on the same patient, comprising a plurality of passive optical markers (4) positionable on the body of the patient and a plurality of active optical markers (5, 6) located on the ecographic probe and on the surgical instrument, sensors of optical signals (8) provided with devices (9) for activation of said passive markers, said sensors (8) being suitable to reception of optical signals produced by reflection of said passive markers and coming from said active markers, a turning on device (11) for said active markers and said devices (9) for the activation of the passive markers, a movement analysis device (10) suitable to process the signals emitted by said sensors (8) as a function of optical signals being received in order to obtain from them the coordinates of said markers, a decoding device (12) suitable to distinguish the coordinates of the active markers from the ones of the passive markers, a data processing device (13) for localization of the ecographic probe (2) and the surgical instrument (3) and for definition of the movement of the patient, a device (14) for acquisition of the ecographic images in synchronism with the position of the ecographic probe (2) and of the surgical instrument (3) and a device (15) for fusion and navigation of the ecographic images with volumetric images coming from other systems (16) for acquisition of images, **characterised in that** said turning on device (11) controls the turning on of the active markers and of the activation devices (9) as an alternative to each other.

2. Apparatus according to claim 1, **characterised in that** active optical markers are also positionable on the body of the patient (1).

3. Apparatus according to claim 1 or 2, **characterised in that** said active markers are made up of high luminosity LED devices.

4. Apparatus according to claim 1, **characterised in that** the markers (4) located on the body of the patient (1), the markers (5) located on the probe (2) and the markers (6) located on the surgical instrument (3) are in numb at least equal to three.

5. Apparatus according to claim 1, **characterised in that** said sensors (8) are made up of video cameras and said activation devices (9) are made up of optical illuminators.

6. Apparatus according to claim 5, **characterised in that** said illuminators (9) are of the infrared light type.

7. Apparatus according to claim 1, **characterised in that** said turning on device (11) controls the turning on of the active markers (5) located on the probe (2) as an alternative to the one of the active markers (6) located on the surgical instrument (3).

8. Apparatus according to claim 1 or 7, **characterised in that** said turning on device (11) comprises a microcontroller (MC) for the control of the turning on of said activation devices (9) and of the active markers and means (P1-P3) to vary the turning on times of said activation devices (9) and said active markers.

9. Apparatus according to claim 1, **characterised in that** said device (10) for the analysis of the movement operates on the basis of photogrammetry algorithms.

10. Apparatus according to claim 1, **characterised in that** said decoding device (12) carries out the calculation of the coordinates of the markers on the basis of stereophotogrammetry algorithms and it utilises procedures for the pursuit of the movements of the markers based on prediction algorithms based on analytical or functional criteria or on Kalman filtering.

11. Apparatus according to claim 1, **characterised in that** said data processing device (13) is capable to identify the parameters of the geometric transformations of the points belonging to the plane of the ecographic image produced by the probe (2) arid to calculate the new co-ordinates of such points in the reference system of the laboratory.

12. Apparatus according to claim 1, **characterised in that** said device (14) for the acquisition of the ecographic images in synchronism with the position of the ecographic probe (2) and/or of the surgical instrument (3) comprises a digitalizer of video signals controlled by a program that receives as an input a series of synchronization signals coming from said device of analysis (10).

13. Apparatus according to claim 1, **characterised in that** said device (15) for fusion and navigation of the ecographic images with volumetric images coming from other systems for the acquisition of images (16) comprises a calculation and visualization program that at each moment of the acquisition of the ecographic images, starting from the geometric parameters coming from said device of acquisition (14) and from the data relative to the position of the patient coming from said processing device (13),

   a) calculates the position of each element of the ecographic image in the reference system of the patient;
   b) it calculates the position of each element of the volumetric image in the reference system of the patient;
   c) it represents on a screen, in a single spacial reference, the volumetric images, the ecographic images and possibly the surgical instrument.

**Patentansprüche**

1. Vorrichtung zur Verschmelzung echografischer und volumetrischer Bilder eines Patienten (1) und zur Navigation und Lokalisierung einer echografischen Sonde (2) und eines Operationsbestecks (3), die an diesem Patienten operieren, mit einer Vielzahl passiver optischer Markierungen (4), die am Körper des Patienten positionierbar sind, und einer Vielzahl aktiver optischer Markierungen (5, 6), die an der echografischen Sonde und an dem Operationsbesteck angeordnet sind, Sensoren für optische Signale (8), die mit Einrichtungen (9) zur Aktivierung der passiven Markierungen versehen sind, wobei die Sensoren (8) für einen Empfang optischer Signale geeignet sind, die durch Reflexion der passiven Markierungen erzeugt sind und von den aktiven Markierungen kommen, einer Einschalteinrichtung (11) für die aktiven Markierungen und die Einrichtungen (9) zur Aktivierung der passiven Markierungen, einer Bewegungsanalyseeinrichtung (10), die zum Verarbeiten der durch die Sensoren (8) emittierten Signale als Funktion optischer Signale geeignet ist, die empfangen werden, um aus ihnen die Koordinaten der Markierungen zu erhalten, einer Decodiereinrichtung (12), die zum Unterscheiden der Koordinaten der aktiven Markierungen von denjenigen der passiven Markierungen geeignet sind, einer Datenverarbeitungseinrichtung (13) zur Lokalisierung der echografischen Sonde (2) und des Operationsbestecks (3) und zur Definition der Bewegung des Patienten, einer Einrichtung (14) zur Erlangung der echografischen Bilder synchron zu der Position der echografischen Sonde (2) und des Operationsbestecks (3) und einer Einrichtung (15) zur Verschmelzung und Navigation der echografischen Bilder mit volumetrischen Bildern, die von anderen Systemen (16) kommen, zur Erlangung von Bildern, **dadurch gekennzeichnet, dass** die Einschalteinrichtung (14) das Einschalten der aktiven Markierungen und der Aktivierungseinrichtungen (9) als Alternative zueinander steuert.

2. Vorrichtung nach Anspruch 1, **dadurch gekennzeichnet, dass** aktive optische Markierungen auch am Körper des Patienten (1) positionierbar sind.

3. Vorrichtung nach Anspruch 1 oder 2, **dadurch gekennzeichnet, dass** die aktiven Markierungen aus LED-Einrichtungen hoher Leuchtkraft gebildet sind.

4. Vorrichtung nach Anspruch 1, **dadurch gekennzeichnet, dass** die am Körper des Patienten (1) lokalisierten Markierungen (4), die an der Sonde (2) lokalisierten Markierungen (5) und die am Operationsbesteck (3) lokalisierten Markierungen (6) anzahlmäßig wenigstens gleich drei sind.

5. Vorrichtung nach Anspruch 1, **dadurch gekennzeichnet, dass** die Sensoren (8) aus Videokameras gebildet sind und die Aktivierungseinrichtungen (9) aus optischen Beleuchtungseinrichtungen gebildet sind.

6. Vorrichtung nach Anspruch 5, **dadurch gekennzeichnet, dass** die Beleuchtungseinrichtungen (9) vom Infrarotlicht-Typ sind.

7. Vorrichtung nach Anspruch 1, **dadurch gekennzeichnet, dass** die Einschalteinrichtung (11) das Einschalten der an der Sonde (2) lokalisierten aktiven Markierungen (5) als Alternative zu demjenigen der am Operationsbesteck (3) lokalisierten aktiven Markierungen (6) steuert.

8. Vorrichtung nach Anspruch 1 oder 7, **dadurch gekennzeichnet, dass** die Einschalteinrichtung (11) eine Mikrosteuerung (MC) für die Steuerung des Einschaltens der Aktivierungseinrichtungen (9) und der aktiven Markierungen und eine Einrichtung (P1-P3) zum Variieren der Einschaltzeiten der Aktivierungseinrichtungen (9) und der aktiven Markierungen aufweist.

9. Vorrichtung nach Anspruch 1, **dadurch gekennzeichnet, dass** die Einrichtung (10) für die Analyse der Bewegung auf der Basis von Photogrammetriealgorithmen arbeitet.

10. Vorrichtung nach Anspruch 1, **dadurch gekennzeichnet, dass** die Decodiereinrichtung (12) die Berechnung der Koordinaten der Markierungen auf der Basis von Stereophotogrammetriealgorithmen ausführt und sie Prozeduren für die Verfolgung der Bewegungen der Markierungen basierend auf Vorhersagealgorithmen basierend auf analytischen oder funktionellen Kriterien oder auf einer Kalman-Filterung verwendet.

11. Vorrichtung nach Anspruch 1, **dadurch gekennzeichnet, dass** die Datenverarbeitungseinrichtung (13) die Parameter der geometrischen Transformationen der Punkte identifizieren, die zu der Ebene des durch die Sonde (2) erzeugten echografischen Bilds gehören, und die neuen Koordinaten von solchen Punkten in dem Bezugssystem des Labors berechnen kann.

12. Vorrichtung nach Anspruch 1, **dadurch gekennzeichnet, dass** die Einrichtung (14) zur Erlangung der echografischen Bilder synchron zu der Position der echografischen Sonde (2) und/oder des Operationsbestecks (3) einen Digitalisierer von Videosignalen aufweist, der durch ein Programm gesteuert wird, das als Eingabe eine Reihe von Synchronisierungssignalen empfängt, die von der Analyseeinrichtung (10) kommen.

13. Vorrichtung nach Anspruch 1, **dadurch gekennzeichnet, dass** die Einrichtung (15) zur Verschmelzung und Navigation der echografischen Bilder mit volumetrischen Bildern, die von anderen Systemen kommen, zur Erlangung von Bildern (16) ein Berechnungs- und Visualisierungsprogramm aufweist, das zu jedem Zeitpunkt der Erlangung der echografischen Bilder beginnend ab den geometrischen Parametern, die von der Erlangungseinrichtung (14) kommen, und ab den Daten in Bezug auf die Position des Patienten, die von der Verarbeitungseinrichtung (13) kommen,

 a) die Position jedes Elements des echografischen Bilds in dem Bezugssystem des Patienten berechnet;
 b) die Position jedes Elements des volumetrischen Bilds in dem Bezugssystem des Patienten berechnet;
 c) auf einem Bildschirm in einer einzigen räumlichen Abbildung die volumetrischen Bilder, die echografischen Bilder und möglicherweise das Operationsbesteck darstellt.

## Revendications

1. Dispositif pour la fusion d'images échographiques et volumétriques d'un patient (1) et pour la navigation et la localisation d'une sonde échographique (2) et d'un instrument chirurgical (3) opérant sur le même patient, comprenant une pluralité de marqueurs optiques passifs (4) pouvant être positionnés sur le corps du patient et une pluralité de marqueurs optiques actifs (5, 6) situés sur la sonde échographique et sur l'instrument chirurgical, des capteurs de signaux optiques (8) pourvus de dispositifs (9) pour l'activation desdits marqueurs passifs, lesdits capteurs (8) étant adaptés pour la réception de signaux optiques produits par la réflexion desdits marqueurs passifs et provenant desdits marqueurs actifs, un dispositif de mise en marche (11) pour lesdits marqueurs actifs et lesdits dispositifs (9) pour l'activation des marqueurs passifs, un dispositif d'analyse de mouvement (10) adapté pour traiter les signaux émis par lesdits capteurs (8) en fonction de signaux optiques reçus afin d'obtenir de ceux-ci les coordonnées desdits marqueurs, un dispositif de décodage (12) adapté pour distinguer les coordonnées des marqueurs actifs de celles des marqueurs passifs, un dispositif de traitement de données (13) pour la localisation de la sonde échographique (2) et de l'instrument chirurgical (3) et pour la définition du mouvement du patient, un dispositif (14) pour l'acquisition des images échographiques en synchronisme avec la position de la sonde échographique (2) et de l'instrument chirurgical (3) et un dispositif (15) pour la fusion et la navigation des images échographiques avec des images volumétriques venant d'autres systèmes (16) pour l'acquisition d'images, **caractérisé en ce que** ledit dispositif de mise en marche (11) commande l'activation des marqueurs actifs et des dispositifs d'activation (9) comme alternative les uns des autres.

2. Dispositif selon la revendication 1, **caractérisé en ce que** des marqueurs optiques actifs peuvent être aussi positionnés sur le corps du patient (1).

3. Dispositif selon la revendication 1 ou 2, **caractérisé en ce que** lesdits marqueurs actifs sont constitués de dispositifs de type DEL à haute luminosité.

4. Dispositif selon la revendication 1, **caractérisé en ce que** les marqueurs (4) situés sur le corps du patient (1), les marqueurs (5) situés sur la sonde (2) et les marqueurs (6) situés sur l'instrument chirurgical (3) sont au nombre d'au moins trois.

5. Dispositif selon la revendication 1, **caractérisé en ce que** lesdits capteurs (8) sont constitués de caméras vidéo et lesdits dispositifs d'activation (9) sont constitués de dispositifs d'éclairage.

6. Dispositif selon la revendication 5, **caractérisé en ce que** lesdits dispositifs d'activation (9) sont du type à lumière infrarouge.

7. Dispositif selon la revendication 1, **caractérisé en ce que** ledit dispositif de mise en marche (11) commande l'activation des marqueurs actifs (5) situés sur la sonde (2) comme alternative à celui des marqueurs actifs (6) situé sur l'instrument chirurgical (3).

8. Dispositif selon la revendication 1 ou 7, **caractérisé en ce que** ledit dispositif de mise en marche (11) comprend un microcontrôleur (MC) pour la commande de l'activation desdits dispositifs d'activation (9) et des marqueurs actifs et un moyen (P1-P3) pour faire varier les temps d'activation desdits dispositifs d'activation (9) et desdits marqueurs actifs.

9. Dispositif selon la revendication 1, **caractérisé en ce que** ledit dispositif (10) pour l'analyse du mouvement fonctionne sur la base d'algorithmes de photogrammétrie.

10. Dispositif selon la revendication 1, **caractérisé en ce que** ledit dispositif de décodage (12) effectue le calcul des coordonnées des marqueurs sur la base d'algorithmes de stéréophotogrammétrie et utilise des procédures pour la poursuite des mouvements des marqueurs basée sur des algorithmes de prédiction basés sur des critères analytiques ou fonctionnels ou sur un filtrage de Kalman.

11. Dispositif selon la revendication 1, **caractérisé en ce que** ledit dispositif de traitement de données (13) est capable d'identifier les paramètres des transformations géométriques des points appartenant au plan de l'image échographique produite par la sonde (2) et de calculer les nouvelles coordonnées de ces points dans le système de référence du laboratoire.

12. Dispositif selon la revendication 1, **caractérisé en ce que** ledit dispositif (14) pour l'acquisition des images échographiques en synchronisme avec la position de la sonde échographique (2) et/ou de l'instrument chirurgical (3) comprend un numériseur de signaux vidéo commandé par un programme qui reçoit en entrée une série de signaux de synchronisation provenant dudit dispositif d'analyse (10).

13. Dispositif selon la revendication 1, **caractérisé en ce que** ledit dispositif (15) pour la fusion et la navigation des images échographiques avec des images volumétriques provenant d'autres systèmes pour l'acquisition d'images (16) comprend un programme de calcul et de visualisation qui à chaque moment de l'acquisition des images échographiques, à partir des paramètres géométriques provenant dudit dispositif d'acquisition (14) et des données relatives à la position du patient provenant dudit dispositif de traitement (13),

> a) calcule la position de chaque élément de l'image échographique dans le système de référence du patient ;
> b) calcule la position de chaque élément de l'image volumétrique dans le système de référence du patient ;
> c) représente sur un écran, dans une seule référence spatiale, les images volumétriques, les images échographiques et éventuellement l'instrument chirurgical.

FIG.1

EP 1 804 705 B1

FIG.2

FIG.3

FIG.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040138556 A

**Non-patent literature cited in the description**

- **Borghese, N. A. ; G. Ferrigno.** An algorithm for 3-D Automatic Movement Detection by means of Standard TV Cameras. *Ieee Trans Biomed Eng,* 1990, vol. 37, 1221-1225
- **Cerveri ; Borghese ; Pedotti.** Complete calibration of a stereo photogrammetric system through control points of unknown coordinates. *Journal of Biomechanics,* 1998, vol. 31, 935-940
- **Tsai.** A versatile camera calibration technique for high accuracy 3D machine vision metrology using off-the-shelf TV cameras and lenses. *IEEE Journal of Robotics and Automation,* 1987, vol. RA-3 (4), 323-344
- **Gamage ; Lasenby.** New least squares solutions for estimating the average center of rotation and the axis of rotation. *Journal of Biomechanics,* 2002, vol. 35, 87-93
- **Cappozzo ; Catani ; Leardini ; Blessed ; Groce.** Position and orientation in space of bones during movement: Experimental artifacts. *Clinical Biomechanics,* 1996, vol. 11, 90-100
- **Brown ; Hwang.** Introduction to random signals and applied Kalman filtering. John Wiley & Sons Inc, 1997
- **Cerveri ; Pedotti ; Ferrigno.** Robust recovery of human motion from video using Kalman filters and virtual humans. *Human Movement Science,* 2003, vol. 22, 377-404
- **Legget et al.** System for quantitative three-dimensional echocardiography of the left ventricle based on a magnetic-field position and orientation sensing system. *IEEE Trans Biomed Eng,* 1998, vol. 45, 494-504
- **Barry et al.** Three-dimensional freehand ultrasound: Image reconstruction and volume analysis. *Ultrasound Med Biol,* 1997, vol. 23, 1209-1224
- **Berg et al.** Dynamic three-dimensional freehand echiocardiography using raw digital ultrasound data. *Ultrasound Med Biol,* 1999, vol. 25, 745-753
- **Bouchet et al.** Calibration of threedimensional ultrasound images for image-guided radiation therapy. *Phys Med Biol,* 2001, vol. 46, 559-577